# DEMANDE DE BREVET EUROPEEN

(11) **EP 1 407 734 A1**
(43) Date de publication de la demande: **14.04.2004**
(21) Numéro de dépôt: 03292293.2
(22) Date de dépôt: 18.09.2003
(51) Int. Cl.: A61F 9/013

(54) **Perfectionnement aux têtes de coupe pour microkeratomes**

(30) Priorité: 07.10.2002 FR 0212410
(71) Demandeur: Moria SA, 92160 Antony (FR)
(72) Inventeur: Aufaure, Jean-Luc, 03210 Souvigny (FR); Frileux, David, 03210 Marigny (FR)
(74) Mandataire: Robert, Jean-Pierre

(57) **Abrégé**

Tête de coupe (1) pour microkératome comportant un logement de guidage (6, 7, 8) pour une lame de coupe oscillante ayant un bord de coupe, un bord arrière et deux bords latéraux, comportant deux paires de faces parallèles écartées d'une distance sensiblement égale à l'épaisseur de la lame et situées respectivement au voisinage du bord de coupe et du bord arrière de la lame, caractérisée en ce que chacune des paires de faces parallèles (7a, 7b, 8a, 8b) est d'une longueur, prise parallèlement au bord de coupe, inférieure à l'écartement des bords latéraux de la lame à leur niveau, diminué de l'amplitude d'oscillation.

## Description

La présente invention concerne les appareils de chirurgie réfractive, appelés microkératomes.

### ARRIERE PLAN DE L'INVENTION

Dans ces appareils, une lame de coupe est animée dans un plan d'un mouvement alternatif à une fréquence relativement élevée. Le plan du mouvement est défini par un logement de guidage dans le corps d'une tête de coupe entre deux surfaces parallèles écartées d'une valeur sensiblement égale à l'épaisseur de la lame.

Ces surfaces sont en réalité divisées en deux paires de surfaces l'une guidant la partie arrière de la lame et l'autre sa partie avant au voisinage du bord de coupe.

La lame, mesurée dans la direction de son fil de coupe, entre ses deux bords latéraux, est plus étroite que le logement. La lame et notamment ses bords latéraux peuvent donc détacher les particules des surfaces de guidage lors du mouvement alternatif et ce, au moment de l'intervention. En effet, le matériau de la lame est en général plus dur que le matériau de la tête, surtout si celle-ci est en matière plastique.

En outre, les surfaces en contact entre la tête et la lame sont importantes et dissipent une énergie sous forme de chaleur qu'il convient de minimiser au maximum.

### OBJET DE L'INVENTION

On se propose par la présente invention de réduire sinon de pallier ces inconvénients.

### BREVE DESCRIPTION DE L'INVENTION

A cet effet, l'invention a pour objet une tête de coupe pour microkératome comportant un logement de guidage pour une lame de coupe oscillante ayant un bord de coupe, un bord arrière et deux bords latéraux, comportant deux paires de faces parallèles écartées d'une distance sensiblement égale à l'épaisseur de la lame et situées respectivement au voisinage du bord de coupe et du bord arrière de la lame, dans laquelle chacune des paires de faces parallèles est d'une longueur, prise parallèlement au bord de coupe, inférieure à l'écartement des bords latéraux de la lame à son niveau, diminué de l'amplitude d'oscillation.

Ainsi, les bords latéraux de la lame de coupe sont-ils « en l'air » sans contact avec les surfaces de guidage, ce qui permet d'éviter la formation de copeaux microscopiques au passage de l'arête de ses bords sur ses surfaces. Bien entendu la position de ces surfaces à l'intérieur de la tête de coupe est centrée autour de la position moyenne de la lame en prise avec un doigt excentrique d'entraînement comme cela est bien connu dans le domaine.

Afin d'éviter l'émission de particules même à l'introduction de la lame, l'extrémité de chaque surface de guidage tournée du côté de la face d'introduction de la lame dans la tête de coupe est chanfreiné.

D'autres caractéristiques et avantages de l'invention ressortiront de la description d'un exemple de sa réalisation donné ci-après à titre indicatif.

### BREVE DESCRIPTION DES DESSINS

Il sera fait référence aux dessins annexés parmi lesquels :
- la figure 1 est une vue de côté d'une tête de microkératome connue,
- la figure 2 est une vue de détail d'une tête de microkératome conforme à la figure 1 équipée des dispositions de l'invention,
- la figure 3 est une vue selon une coupe III-III de la figure 2,
- la figure 4 est une vue selon la coupe IV-IV de la figure 2.

### DESCRIPTION DETAILLEE DE L'INVENTION

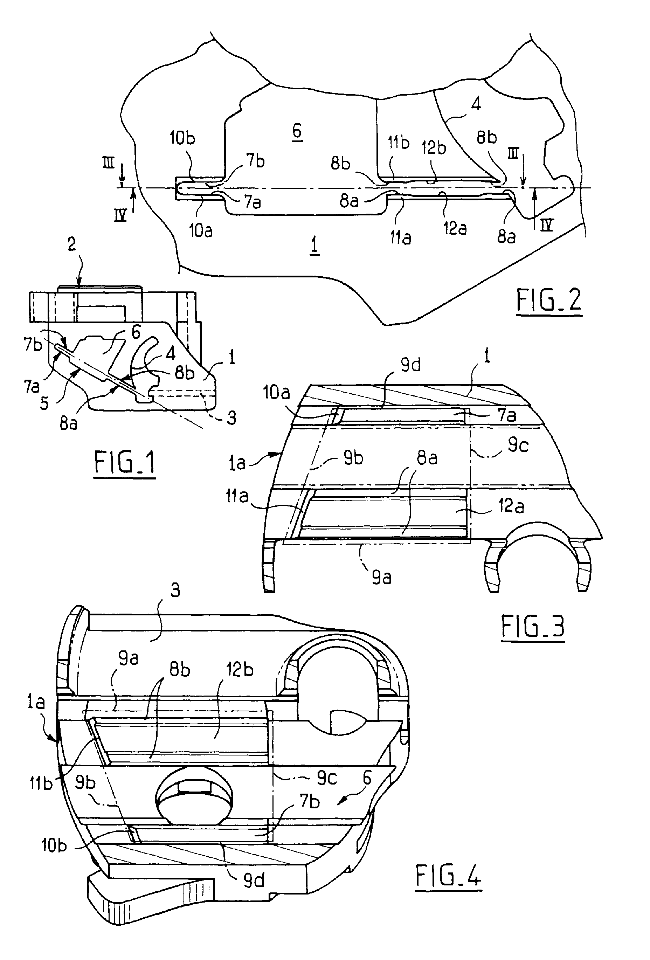

A la figure 1 on a représenté de profil une tête de coupe 1 d'un kératome connu qui possède une face 2 pour son attelage à un groupe moteur, un plateau avant aplanateur 3, un volet déflecteur 4 pour un capot cornéen en cours de découpe et un logement 5 pour le guidage d'une lame de coupe non représentée dans un mouvement alternatif perpendiculaire au plan de la figure. Ce logement 5 comporte une partie centrale 6 dans laquelle évolue le talon d'entraînement ou navette de la lame. Cette partie centrale est bordée par une première paire 7a, 7b de surfaces parallèles espacées sensiblement de l'épaisseur de la lame et formant guidage de celle-ci du côté de son bord arrière et une seconde paire de surfaces parallèles 8a, 8b, espacées de la même manière et formant guidage de sa partie voisine de son bord de coupe situé au voisinage du bord arrière du plateau d'aplanation 3.

Aux figures 2, 3 et 4, on retrouve certains des éléments déjà décrits avec les mêmes références, sur une tête de kératome comportant les dispositions de l'invention.

Aux figures 3 et 4, on a représenté la lame de coupe 9 en trait mixte, dans sa position moyenne en service. Cette lame comporte un bord de coupe 9a, deux bords latéraux 9b, 9c, et un bord arrière 9d. On constate que les surfaces 7a, 7b de guidage du bord arrière de la lame sont de longueur inférieure à la distance séparant à ce niveau.l'écartement des bords latéraux 9b et 9c. Cette longueur pourra être très nettement inférieure à cet écartement et ne sera pas supérieure à cet écartement diminué de l'amplitude d'oscillation de la lame dans son plan de guidage. On constate que du côté 1a d'introduction de la lame dans la tête 1, les faces 7a et 7b possèdent des chanfreins 10a, 10b formant rampe de guidage du bord 9c de la lame. Pour ce qui concerne les faces 8a et 8b, on constate que leur longueur répond aux même critères que ceux 7a et 7b par rapport à la lame 9, qu'elles possèdent également un chanfrein 11a, 11b pour l'introduction de cette lame et qu'en plus elles possèdent un dégagement central 12a, 12b qui réduit la surface frottante entre la tête de coupe et la lame.

Les dispositions de l'invention trouvent une application dans tous les types de microkératomes, qu'ils soient motorisés ou à avance manuelle, à course de coupe rectiligne ou circulaire.

## Revendications

1. Tête de coupe (1) pour microkératome comportant un logement de guidage (6, 7, 8) pour une lame de coupe oscillante ayant un bord de coupe, un bord arrière et deux bords latéraux, comportant deux paires de faces parallèles écartées d'une distance sensiblement égale à l'épaisseur de la lame et situées respectivement au voisinage du bord de coupe et du bord arrière de la lame, **caractérisée en ce que** chacune des paires de faces parallèles (7a, 7b, 8a, 8b) est d'une longueur, prise parallèlement au bord de coupe, inférieure à l'écartement des bords latéraux de la lame à leur niveau, diminué de l'amplitude d'oscillation.

2. Tête de coupe selon la revendication 1, **caractérisée en ce que** l'extrémité de chaque face de guidage (7a, 7b, 8a, 8b) tournée du côté de la face (1a) de la tête d'introduction de la lame dans celle-ci, est chanfreinée (10a, 10b, 11a, 11b).

3. Tête de coupe selon la revendication 1 ou la revendication 2, **caractérisée en ce que** le profil de chacune des faces parallèles (8a, 8b) de la paire de faces voisines du bord de coupe de la lame présente un dégagement central (12a, 12b).
